# FASCICULE DE BREVET EUROPEEN

(11) **EP 0 858 882 B1**
(45) Date de publication et mention de la délivrance du brevet: **29.10.2003**
(21) Numéro de dépôt: 98200251.1
(22) Date de dépôt: 28.01.1998
(51) Int. Cl.: B32B 27/08, B32B 1/08, A61L 29/00

(54) **Tube comprenant au moins 3 couches**
Wenigstens drei Schichten enthaltender Schlauch
Tube comprising at least three layers

(30) Priorité: 11.02.1997 BE 9700126
(43) Date de publication de la demande: 19.08.1998
(73) Titulaire: SOLVAY (Société Anonyme), 1050 Bruxelles (BE)
(72) Inventeur: Karsten, Petrus J.A., 1602 DL Enkhuizen (NL)
(74) Mandataire: Jacques, Philippe

(56) Documents cités:
- US-A- 5 532 053

## Description

La présente invention concerne un tube multicouche, pouvant notamment être avantageusement utilisé dans des applications médicales, dont les exigences sont très élevées.

Plusieurs polymères spécifiques ont déjà été proposé pour satisfaire ces exigences. Ainsi, par exemple, on a déjà proposé d'utiliser des copolyesteréthers fabriqués à partir de poly(tétraméthylène oxyde)glycol, d'acide 1,4-cyclohexane-dicarboxylique et de 1,4-cyclohexane-diméthanol. La fabrication de tels copolymères (appelés "PCCE" ci-après) est notamment décrite dans le brevet US 4349469.

Les exigences imposées aux tubes à usage médical sont notamment des exigences de souplesse, d'atoxicité ("medical grade"), de transparence et de soudabilité haute-fréquence (HF). La fabrication de tels tubes pose dès lors des problèmes très spécifiques, ce qui fait que des polymères convenant par exemple à la fabrication de films ne conviennent pas forcément à la fabrication de tubes.

Ainsi, les PCCE, qui conviennent à la fabrication de films, se prêtent mal à la fabrication de tubes en raison de leur cristallisation rapide, qui affecte négativement la transparence et la souplesse. Un refroidissement brusque (trempe) peut certes contribuer à atténuer ces problèmes, mais cette solution n'est efficace que pour des épaisseurs extrêmement faibles, par exemple de moins de 200 µm. Une trempe ne constituerait ainsi une solution satisfaisante que dans la fabrication de tubes extrêmlement minces, mais n'est pas suffisante à elle seule lorsqu'on envisage de fabriquer des tubes à usage médical, qui ont généralement une épaisseur d'au moins 500 µm. En outre, les PCCE sont relativement peu polaires, et se prêtent donc mal au soudage HF.

La présente invention vise à remédier à ces problèmes, et à fournir un tube qui soit souple, transparent, soudable HF et atoxique.

Plus précisément, la présente invention concerne un tube comprenant :
(a) une couche intérieure essentiellement constituée d'un copolyester obtenu à partir d'au moins un poly(alkylène oxyde)glycol, d'acide 1,4-cyclohexane-dicarboxylique et de 1,4-cyclohexane-diméthanol ,
(b) une couche intermédiaire essentiellement constituée de copolymère éthylène-acétate de vinyle et/ou d'un copolymère comprenant au moins un bloc de polyamide et au moins un bloc de poly(alkylène oxyde) ;
(c) une couche extérieure essentiellement constituée d'un copolyester obtenu à partir d'au moins un poly(alkylène oxyde)glycol, d'acide 1,4-cyclohexane-dicarboxylique et de 1,4-cyclohexane-diméthanol.

Avantageusement, la couche intérieure (a) présente une épaisseur n'excédant pas 300 µm, et de préférence pas 200 µm, ce qui lui permet de conserver une souplesse et une transparence satisfaisantes. Son épaisseur est par ailleurs généralement d'au moins 50 µm.

Le polymère constitutif de la couche (a) est un copolyester comprenant un ou plusieurs blocs de type polyéther (poly(alkylène oxyde)), qui peuvent être obtenus par la polymérisation préalable de composés du type alkylène glycol (par exemple tétraméthylène glycol) ou éther cyclique (par exemple tétrahydrofurane), et qui sont ensuite copolymérisés avec les constituants susmentionnés sous la forme de poly(alkylène oxyde)glycols. Le poly(alkylène oxyde) du copolyester de la couche (a) peut être de tout type connu, par exemple du poly(propylène oxyde) ou du poly(tétraméthylène oxyde). On préfère que le poly(alkylène oxyde) du copolyester de la couche (a) soit du poly(tétraméthylène oxyde). Outre les trois réactifs de départ susmentionnés (poly(alkylène oxyde)glycol, acide 1,4-cyclohexane-dicarboxylique et 1,4-cyclohexane-diméthanol), le copolyester de la couche (a) peut également être synthétisé en présence d'une quantité modérée d'un ou plusieurs agents de branchement tels que par exemple des triacides ou des trialcools, permettant d'obtenir des chaînes polymériques ramifiées. La couche (a) peut naturellement comprendre plusieurs copolyesters de ce type.

L'épaisseur de la couche intermédiaire (b) peut être ajustée en fonction des exigences spécifiques de résistance mécanique, de souplesse, de transparence, de soudabilité, etc. du tube. De bons résultats ont été obtenus en donnant à la couche (b) une épaisseur de 100 à 600 µm. Si toutefois la couche (b) est constituée de copolymère éthylène-acétate de vinyle (EVA), il est préférable de limiter son épaisseur à 300 µm, ceci afin d'éviter d'éventuels problèmes en cas de stérilisation à la vapeur. On notera que le choix du polymère constitutif de la couche intérieure n'est pas trivial ; ainsi, des essais d'extrusion de tubes tricouches PCCE/PVC/PCCE ont conduit à une sévère dégradation du PVC constitutif de la couche intérmédiaire.

Lorsque la couche (b) est constituée de copolymère éthylène-acétate de vinyle, il est avantageux que la teneur de celui-ci en acétate de vinyle soit de l'ordre de 10 à 40 % en poids.

Lorsque la couche (b) est constituée de copolymère (couramment appelé "polyester block amide", ou en abrégé "PEBA") comprenant au moins un bloc de polyamide et au moins un bloc de poly(alkylène oxyde), il est avantageux que ce dernier soit un bloc de poly(tétraméthylène oxyde). Dans ce cas, de bons résultats ont par ailleurs été obtenus en utilisant spécifiquement un copolymère comprenant au moins un bloc de polyamide 12 et au moins un bloc de poly(alkylène oxyde). La couche (b) peut naturellement comprendre plusieurs polymères de ces types.

L'épaisseur de la couche (c) est de préférence de 50 à 400 µm. La couche extérieure étant plus facile à refroidir que la couche intérieure, ceci autorise en effet des épaisseurs supérieures. Selon une variante avantageuse, le poly(alkylène oxyde) du copolyester de la couche (c) est du poly(tétraméthylène oxyde). La couche (c) peut naturellement comprendre plusieurs polymères du type décrit ci-dessus.

Outre les polymères susdéfinis, les couches (a), (b) et/ou (c) peuvent encore comprendre des quantités modérées d'un ou plusieurs additifs usuels, tels qu'antioxydants, stabilisants, pigments, etc., pour autant que cela ne nuise pas aux propriétés finales du tube.

Outre les couches (a), (b) et (c), le tube peut encore contenir une ou plusieurs autres couches, pour autant qu'elles ne nuisent pas aux propriétés souhaitées. En particulier, afin d'optimiser l'adhérence mutuelle des différentes couches susmentionnées, on peut éventuellement leur interposer une ou plusieurs couches d'adhésifs. En vue de se passer de tels adhésifs, une variante avantageuse consiste ce que la couche (b) soit essentiellement constituée d'un copolymère comprenant au moins un bloc de polyamide et au moins un bloc de poly(alkylène oxyde), et en ce que le même poly(alkylène oxyde) soit utilisé dans les polymères constitutifs des couches (a), (b) et (c).

De très bons résultats ont été obtenus avec des tubes dans lesquels les 3 couches (a), (b) et (c) présentent les épaisseurs suivantes : (a) de 50 à 300 µm, (b) de 100 à 600 µm, (c) de 50 à 400 µm.

Le tube de l'invention est avantageusement fabriqué par un procédé de coextrusion. De préférence, immédiatement après l'extrusion du tube, on le soumet à un refroidissement rapide (trempe) en vue d'en améliorer la flexibilité et la transparence.

Le tube de l'invention peut naturellement être utilisé dans des applications autres que médicales.

Il se prête toutefois particulièrement bien à des utilisations dans le domaine médical. Sa bonne soudabilité HF permet de le souder aisément à des récipients souples tels que des poches destinées à contenir du sang ou d'autres liquides à usage médical. A cette fin, la présente invention concerne également un dispositif de distribution de produits à usage médical - en particulier une poche à sang - comprenant au moins une poche souple à base de matière thermoplastique à laquelle est soudée au moins un tube tel que décrit ci-dessus. Le soudage est avantageusement effectué par soudage HF. L'invention concerne également un procédé de fabrication d'un tel dispositif de distribution, impliquant une étape de soudage HF.

## Revendications

1. Tube comprenant :
(a) une couche intérieure essentiellement constituée d'un copolyester obtenu à partir d'au moins un poly(alkylène oxyde)glycol, d'acide 1,4-cyclohexane-dicarboxylique et de 1,4-cyclohexane-diméthanol ;
(b) une couche intermédiaire essentiellement constituée de copolymère éthylène-acétate de vinyle et/ou d'un copolymère comprenant au moins un bloc de polyamide et au moins un bloc de poly(alkylène oxyde) ;
(c) une couche extérieure essentiellement constituée d'un copolyester obtenu à partir d'au moins un poly(alkylène oxyde)glycol, d'acide 1,4-cyclohexane-dicarboxylique et de 1,4-cyclohexane-diméthanol.

2. Tube selon la revendication 1, dans lequel le poly(alkylène oxyde) du copolyester de la couche (a) est du poly(tétraméthylène oxyde).

3. Tube selon l'une des revendications précédentes, dans lequel la couche (b) est essentiellement constituée d'un copolymère comprenant au moins un bloc de polyamide et au moins un bloc de poly(tétraméthylène oxyde).

4. Tube selon l'une des revendications précédentes, dans lequel la couche (b) est essentiellement constituée d'un copolymère comprenant au moins un bloc de polyamide 12 et au moins un bloc de poly(alkylène oxyde).

5. Tube selon l'une des revendications précédentes, dans lequel le poly(alkylène oxyde) du copolyester de la couche (c) est du poly(tétraméthylène oxyde).

6. Tube selon l'une des revendications précédentes, dans lequel la couche (b) est essentiellement constituée d'un copolymère comprenant au moins un bloc de polyamide et au moins un bloc de poly(alkylène oxyde), et dans lequel le même poly(alkylène oxyde) est utilisé dans les polymères constitutifs des couches (a), (b) et (c).

7. Tube selon l'une des revendications précédentes, dans lequel la couche (a) présente une épaisseur de 50 à 300 µm.

8. Tube selon l'une des revendications précédentes, dans lequel la couche (b) présente une épaisseur de 100 à 600 µm.

9. Tube selon l'une des revendications précédentes, dans lequel la couche (c) présente une épaisseur de 50 à 400 µm.

10. Dispositif de distribution de produits à usage médical comprenant au moins une poche souple à base de matière thermoplastique à laquelle est soudée au moins un tube selon l'une des revendications précédentes.

## Patentansprüche

1. Schlauch, der umfaßt:
(a) eine Innenschicht, im wesentlichen bestehend aus einem Copolyester, erhalten aus wenigstens einem Poly(alkylenoxid)glycol, 1,4-Cyclohexandicarbonsäure und 1,4-Cyclohexan-dimethanol;
(b) eine Zwischenschicht, im wesentlichen bestehend aus einem EthylenVinylacetat-Copolymer und/oder einem Copolymer, das wenigstens einen Polyamidblock und wenigstens einen Poly(alkylenoxid)block umfaßt;
(c) eine Außenschicht, im wesentlichen bestehend aus einem Copolyester, erhalten aus wenigstens einem Poly(alkylenoxid)glycol, 1,4-Cyclohexandicarbonsäure und 1,4-Cyclohexan-dimethanol.

2. Schlauch nach Anspruch 1, worin das Poly(alkylenoxid) des Copolyesters der Schicht (a) Poly(tetramethylenoxid) ist.

3. Schlauch nach einem der vorstehenden Ansprüche, worin die Schicht (b) im wesentlichen aus einem Copolymer besteht, das wenigstens einen Polyamidblock und wenigstens einen Poly(tetramethylenoxid)block umfaßt.

4. Schlauch nach einem der vorstehenden Ansprüche, worin die Schicht (b) im wesentlichen aus einem Copolymer besteht, das wenigstens einen Polyamid 12-Block und wenigstens einen Poly(alkylenoxid)block umfaßt.

5. Schlauch nach einem der vorstehenden Ansprüche, worin das Poly(alkylenoxid) des Copolyesters der Schicht (c) Poly(tetramethylenoxid) ist.

6. Schlauch nach einem der vorstehenden Ansprüche, worin die Schicht (b) im wesentlichen aus einem Copolymer besteht, das wenigstens einen Polyamidblock und wenigstens einen Poly(alkylenoxid)block umfaßt und worin das gleiche Poly(alkylenoxid) in den die Schichten (a), (b) und (c) ausbildenden Polymeren verwendet wird.

7. Schlauch nach einem der vorstehenden Ansprüche, worin die Schicht (a) eine Stärke von 50 bis 300 um aufweist.

8. Schlauch nach einem der vorstehenden Ansprüche, worin die Schicht (b) eine Stärke von 100 bis 600 µm aufweist.

9. Schlauch nach einem der vorstehenden Ansprüche, worin die Schicht (c) eine Stärke von 50 bis 400 µm aufweist.

10. Vorrichtung zur Abgabe von Produkten zur medizinischen Verwendung, umfassend wenigstens einen flexiblen Beutel auf der Basis eines thermoplastischen Materials, an den wenigstens ein Schlauch nach einem der vorstehenden Ansprüche angeschweißt ist.

## Claims

1. Tube comprising:
(a) an inner layer essentially consisting of a copolyester obtained from at least one poly(alkylene oxide) glycol, 1,4-cyclbhexanedicarboxylic acid and 1,4-cyclohexanedimethanol;
(b) an interlayer essentially consisting of an ethylene-vinyl acetate copolymer and/or of a copolymer containing at least one polyamide block and at least one poly(alkylene oxide) block; and
(c) an outer layer essentially consisting of a copolyester obtained from at least one poly(alkylene oxide) glycol, 1,4-cyclohexanedicarboxylic acid and 1,4-cyclohexanedimethanol.

2. Tube according to Claim 1, in which the poly(alkylene oxide) of the copolyester of layer (a) is poly(tetramethylene oxide).

3. Tube according to one of the preceding claims, in which layer (b) essentially consists of a copolymer containing at least one polyamide block and at least one poly(tetramethylene oxide) block.

4. Tube according to one of the preceding claims, in which layer (b) essentially consists of a copolymer containing at least one nylon-12 block and at least one poly(alkylene oxide) block.

5. Tube according to one of the preceding claims, in which the poly(alkylene oxide) of the copolyester of layer (c) is poly(tetramethylene oxide).

6. Tube according to one of the preceding claims, in which layer (b) essentially consists of a copolymer containing at least one polyamide block and at least one poly(alkylene oxide) block and in which the same poly(alkylene oxide) is used in the polymers making up layers (a), (b) and (c).

7. Tube according to one of the preceding claims, in which layer (a) has a thickness of 50 to 300 µm.

8. Tube according to one of the preceding claims, in which layer (b) has a thickness of 100 to 600 µm.

9. Tube according to one of the preceding claims, in which layer (c) has a thickness of 50 to 400 µm.

10. Device for dispensing products for medical use, comprising at least one thermoplastic-based flexible bag to which at least one tube according to one of the preceding claims is welded.
